# EUROPÄISCHE PATENTSCHRIFT

(11) **EP 0 989 656 B1**
(45) Veröffentlichungstag und Bekanntmachung des Hinweises auf die Patenterteilung: **11.03.2009**
(21) Anmeldenummer: 99810553.0
(22) Anmeldetag: 22.06.1999
(51) Int. Cl.: H02K 7/09

(54) **Permanentmagnetisch erregter elektrischer Drehantrieb**
Permanent magnet excited electric rotary drive
Entraînement de rotation à excitation par aimants permanents

(30) Priorität: 24.09.1998 EP 98810956
(43) Veröffentlichungstag der Anmeldung: 29.03.2000
(73) Patentinhaber: Levitronix LLC, Waltham, MA 02451 (US)
(72) Erfinder: Schöb, Reto, Dr., 8604 Volketswill (CH)
(74) Vertreter: Sulzer Management AG

(56) Entgegenhaltungen:
- WO-A-98/11650
- GB-A- 2 276 050
- US-A- 5 491 622
- US-A- 5 578 880
- PATENT ABSTRACTS OF JAPAN vol. 018, no. 681 (E-1649), 21. Dezember 1994 (1994-12-21) & JP 06 269144 A (SHINKO ELECTRIC CO LTD), 22. September 1994 (1994-09-22)
- PATENT ABSTRACTS OF JAPAN vol. 018, no. 434 (E-1592), 12. August 1994 (1994-08-12) & JP 06 133493 A (SHINKO ELECTRIC CO LTD), 13. Mai 1994 (1994-05-13)
- PATENT ABSTRACTS OF JAPAN vol. 096, no. 007, 31. Juli 1996 (1996-07-31) & JP 08 084491 A (EBARA CORP), 26. März 1996 (1996-03-26)
- PATENT ABSTRACTS OF JAPAN vol. 017, no. 003 (E-1301), 6. Januar 1993 (1993-01-06) & JP 04 236188 A (TOSHIBA CORP), 25. August 1992 (1992-08-25)

## Beschreibung

Die Erfindung betrifft einen permanentmagnetisch erregten elektrischen Drehantrieb für eine Blutpumpe gemäss dem Oberbegriff des unabhängigen Anspruchs sowie eine Blutpumpe mit einem solchen Drehantrieb.

Blutpumpen, die üblicherweise als Axial- oder als Zentrifugalpumpen ausgestaltet sind, dienen der Förderung von Blut und werden beispielsweise im Rahmen von Operationen am Herzen zur Aufrechterhaltung des Blutkreislaufs eingesetzt. Ferner sind implantierbare Blutpumpen bekannt, die zur temporären oder chronischen Unterstützung der Herztätigkeit in den Körper des Patienten implantiert werden.

Bei Blutpumpen muss es gewährleistet sein, dass es zu keiner Verunreinigung des geförderten Bluts kommt. Daher wird in Blutpumpen der Rotor des elektromagnetischen Antriebs und/oder der Pumpenrotor vorzugsweise magnetisch berührungslos gelagert. Diese magnetische Lagerung des Rotors kann entweder durch separate, das heisst vom Antrieb verschiedene Magnetlager realisiert werden, oder die magnetische Lagerung wird durch den Stator des Antriebs realisiert.

In der WO-A-96/31934 wird beispielsweise eine als Blutpumpe geeignete Rotationspumpe offenbart, die als sogenannter lagerloser Motor ausgestaltet ist, das heisst als elektromagnetischer Drehantrieb mit magnetisch berührungslos gelagertem Rotor, wobei keine separaten Magnetlager für den Rotor vorhanden sind. Der Stator ist dazu als Lager- und Antriebsstator ausgestaltet, der eine Antriebswicklung und eine Steuerwicklung umfasst. Mit diesen beiden Wicklungen lässt sich ein magnetisches Drehfeld erzeugen, welches zum einen ein Drehmoment auf den Rotor ausübt, und welches zum anderen eine beliebig einstellbare Querkraft auf den Rotor ausübt, sodass dessen radiale Position aktiv steuerbar ist.

Weiterhin sollen Blutpumpen, insbesondere im Falle einer Implantation in den Körper, kompakt und platzsparend sein, aber dennoch eine Pumpleistung erbringen können, die zumindest derjenigen des Herzens entspricht. Hierzu wird z. B. in der WO-A-96/31934 vorgeschlagen, den Rotor des lagerlosen Motors mit Flügeln zu versehen, sodass der Rotor des Drehantriebs identisch mit dem Pumpenrotor ist, also einen Integralrotor bildet. Dieser Rotor dient somit als Antriebsrotor, Lagerrotor und Pumpenrotor, wodurch eine sehr kompakte und leistungsfähige Blutpumpe realisierbar ist.

Es sind aber auch Blutpumpen bekannt, bei denen der Pumpenrotor mit welchem das Blut gefördert wird, vom Rotor des Drehantriebs verschieden ist. Der Pumpenrotor ist als Flügel- oder Laufrad ausgestaltet, welches vom Rotor des Drehantriebs in Rotation versetzt wird. Dazu können solche Blutpumpen beispielsweise nach dem Prinzip des Spaltrohrmotors bzw. der Spaltrohrpumpe ausgestaltet sein, oder der Pumpenrotor kann magnetisch an den Rotor des Drehantriebs gekoppelt sein.

Eine wesentliche Bedeutung, insbesondere im Hinblick auf implantierbare Blutpumpen, kommt der Betriebssicherheit zu. Ein Problem bei bekannten Drehantrieben für Blutpumpen ist es, dass beim Auftreten von Fehlern, wie beispielsweise dem Ausfall einer Verstärkerstufe oder dem Bruch einer elektrischen Leitung in einer der Phasen der Antriebswicklung des Stators, ein ordnungsgemässes Funktionieren des Antriebs nicht mehr gewährleistet ist. Ein daraus resultierendes Versagens des Antriebs einer Blutpumpe kann jedoch sehr schlimme, eventuell sogar tödliche Folgen haben kann. Die Erfindung widmet sich daher der Aufgabe, dieses Sicherheitsrisiko deutlich zu reduzieren.

Der Erfindung liegt deshalb die Aufgabe zugrunde, einen elektrischen Drehantrieb für eine Blutpumpe bereitzustellen, der auch beim Auftreten von Fehlern noch einen ordnungsgemässen Betrieb, das heisst insbesondere einen zuverlässigen Antrieb des Rotors, ermöglicht.

Der diese Aufgabe lösende elektrische Drehantrieb für eine Blutpumpe ist durch die Merkmale des unabhängigen Anspruchs gekennzeichnet.

Erfindungsgemäss wird also ein permanentmagnetisch erregter elektrischer Drehantrieb für eine Blutpumpe vorgeschlagen, mit einem permanentmagnetischen Rotor und einem Stator, welcher Stator eine mindestens zwei Stränge aufweisende Antriebswicklung zum Erzeugen eines magnetischen Antriebsfelds umfasst, das ein Drehmoment auf den Rotor bewirkt, wobei jeder Strang zu einer anderen elektrischen Phase gehört, ferner mit einer Stelleinrichtung, die jeden Strang jeweils mit einem Phasenstrom oder jeweils mit einer Phasenspannung als Stellgrösse versorgt, wobei die Stelleinrichtung für jeden Strang einen separaten Leistungsverstärker umfasst, sodass die Stellgrösse für jeden Strang unabhängig von der Stellgrösse für die anderen Stränge regelbar ist, wobei ein belastbarer Sternpunkt vorgesehen ist, der mit jedem Strang der Antriebswicklung verbunden ist und über eine elektrische Leitung direkt mit einem stets gleichen Potential verbunden ist.

Da die Stellgrösse, das heisst die Phasenspannungen oder die Phasenströme, für jeden Strang der Antriebswicklung unabhängig von den Stellgrössen für die anderen Stränge regelbar ist und zudem für jeden Strang ein eigener Leistungsverstärker vorgesehen ist, ist jede elektrische Phase, womit jeweils ein Strang der Antriebswicklung und der ihn versorgenden Teil der Stelleinrichtung gemeint ist, unabhängig von den übrigen elektrischen Phasen betreibar. Somit kann der Drehantrieb beim Auftreten eines Fehlers in einer Phase, z. B. beim Ausfall einer kompletten Phase, mit einer reduzierten Anzahl von Phasen weiter betrieben werden, ohne dass Zugeständnisse an das ordnungsgemässe Funktionieren des Drehantriebs vonnöten sind. Da der erfindungsgemässe Drehantrieb mit einer reduzierten Anzahl von Phasen betrieben werden kann, ist es grundsätzlich egal, wo in einer Phase ein Fehler auftritt. So kann beispielsweise ein Leistungsverstärker ausfallen, oder es kann zum Bruch einer Leitung in einem Strang der Antriebswicklung oder in der Verbindung zwischen Leistungsverstärker und einem Strang der Antriebswicklung kommen, oder es kann ein Kurzschluss in einem Leistungsverstärker oder einem Wicklungsstrang der Antriebswieklung auftreten, und trotz eines solchen Fehlers ist ein weiterer Betrieb des Drehantriebs möglich. Aufgrund dieser hohen Fehlertoleranz bringt der erfindungsgemässe Drehantrieb eine erhebliche Steigerung der Betriebssicherheit mit sich.

Je nachdem, mit wievielen Phasen der erfindungsgemässe Drehantrieb ausgestaltet ist, kann er sogar beim Ausfall mehrere Phasen noch weiter betrieben werden. Die minimale Anforderung für die Funktionstüchtigkeit des Drehantriebs ist es, dass noch eine Phase, also ein Strang der Antriebswicklung und der ihn versorgende Leistungsverstärker, fehlerfrei arbeitet.

Der erfindungsgemässe Drehantrieb mit seiner im fehlerfreien Normalfall mindestens zweiphasigen Ausgestaltung ist ein permanentmagnetisch erregter Drehfeldmotor, also insbesondere ein permanentmagnetisch erregter Synchronmotor oder ein bürstenloser Gleichstrommotor (letzterer ist trotz seines allgemein üblichen Namens dem Wesen nach ein Drehfeldmotor). Das heisst, das vom Stator erzeugte Antriebsfeld ist ein magnetisches Drehfeld, welches den permanentmagnetischen Rotor antreibt. Falls durch einen Fehler in einer Phase oder mehreren Phasen nur noch eine fehlerfreie Phase für den Betrieb zur Verfügung steht, so wird der Drehfeldmotor zu einem einphasigen Wechselstrommotor.

Da im allgemeinen für Blutpumpen und insbesondere für implantierte Blutpumpen kein beliebig grosser Energievorrat zu Verfügung steht, ist es wichtig, dass der Drehantrieb einer Blutpumpe möglichst wirtschaftlich und mit möglichst geringem Energieverbrauch arbeitet. Da der erfindungsgemässe Drehantrieb permanentmagnetisch erregt ist, also einen permanentmagnetisch erregten Rotor aufweist, ist er im Unterschied zu felderregten Drehantrieben besonders gut für Blutpumpen geeignet, denn in einem permanentmagnetisch erregten Drehantrieb wird kein Strom und damit keine Energie für die Felderregung benötigt.

Bei dem erfindungsgemässen Drehantriebs ist ein belastbarer Sternpunkt vorgesehen, der mit jedem Strang der Antriebswicklung verbunden ist. Bei üblichen mehrphasigen, z. B. dreiphasigen Antriebswicklungen sind die drei Stränge jeweils mit einem gemeinsamen Sternpunkt verbunden, wobei die Bedingung erfüllt sein muss, dass die Summe der Phasenströme im Sternpunkt stets Null ist. Durch die Massnahme, den Sternpunkt belastbar zu machen, das heisst ihn auf ein belastbares Potential zu legen, kann diese Bedingung aufgehoben werden, sodass jeder Phasenstrom bzw. jede Phasenspannung unabhängig von den anderen regelbar ist.

Vorzugsweise sind bei diesem Ausführungsbeispiel zwei stabilisierte Spannungsquellen zur Versorgung der Stelleinrichtung vorgesehen, wobei die Spannungsquellen einen gemeinsamen Pol aufweisen und wobei der belastbare Sternpunkt auf dem gleichen Potential liegt wie der gemeinsame Pol der Spannungsquellen. Der gemeinsame Pol kann beispielsweise auf Erdpotential liegen. Die eine Spannungsquelle liefert dann eine positive Versorgungsspannung und die andere Spannungsquelle eine negative Versorgungsspannung.

In einem zweiten Ausführungsbeispiel das der Erläuterung dient und nicht Gegenstand der Erfindung ist weist jeder Strang der Antriebswicklung zwei elektrische Anschlussleitungen auf, wobei die Anschlussleitungen eines Strangs jeweils getrennt und unabhängig von den übrigen Anschlussleitungen sind. Jeder Leistungsverstärker ist dann mit genau zwei Anschlussleitungen verbunden, wobei diese beiden Anschlussleitungen zum selben Strang gehören. Bei diesem Ausführungsbeispiel werden also für jeden Strang der Antriebswicklung zwei Anschlussleitungen bis zu dem Leistungsverstärker geführt, der diesen Strang versorgt. Es existiert also kein gemeinsamer Verbindungspunkt, wie z. B. ein Sternpunkt, an dem Anschlussleitungen, die zu unterschiedlichen Strängen gehören, zusammengeführt bzw. elektrisch leitend verbunden werden. Auch durch diese Massnahme ist es möglich, die Stellgrösse (Phasenstrom oder Phasenspannung) für jeden Strang unabhängig von der Stellgrösse für die anderen Stränge zu regeln.

Aus praktischen Gründen ist es dabei bevorzugt, dass jeder Leistungsverstärker als H-Brückenschaltung ausgestaltet ist.

Eine weitere vorteilhafte Massnahme besteht darin, ein Überwachungsmodul vorzusehen, welches beim Auftreten eines Fehlers in einer der elektrischen Phasen diese deaktiviert. Somit kann nämlich ein unnötiger Energieverbrauch in einer fehlerbehafteten Phase, der beispielsweise durch einen Kurzschlussstrom verursacht werden kann, vermieden werden.

Insbesondere ist es vorteilhaft, für jede elektrische Phase eine Überstromsicherung vorzusehen, damit beim Auftreten eines Kurzschlusses die zugehörige Phase abgeschaltet wird. Somit lässt sich eine hoch unsymmetrische Belastung der Energiequelle, welche die Leistungsverstärker speist, vermeiden.

Der erfindungsgemässe Drehantrieb kann insbesondere auch als lagerloser Motor (in dem vorne erläuterten Sinne) ausgebildet sein, der den Rotor des Drehantriebs magnetisch lagert, wobei der Stator als Antriebs- und Lagerstator ausgestaltet ist, der ausser der Antriebswicklung ferner eine Steuerwicklung umfasst.

Durch die Erfindung wird ferner eine Blutpumpe mit einem erfindungsgemässen permanentmagnetisch erregten elektrischen Drehantrieb vorgeschlagen. Durch die Fehlertoleranz des Drehantriebs zeichnet sich eine solche Blutpumpe durch eine sehr hohe Betriebssicherheit aus, sodass sie insbesondere auch für die Implantation in einen Körper verwendet werden kann. Insbesondere in Kombination mit einer fehlertoleranten magnetischen Lagervorrichtung für den Rotor ist eine Blutpumpe realisierbar, die - wenn überhaupt - nur noch mit einem minimalen Sicherheitsrisiko behaftet ist.

Um die Blutpumpe besonders kompakt auszugestalten, kann der Rotor des Drehantriebs als Pumpenrotor zum Fördern des Bluts ausgestaltet sein, das heisst in sinngemäss gleicher Weise wie dies z. B. für den Integralrotor in der bereits zitierten WO-A-96/31934 offenbart ist, ist der Rotor des Drehantriebs identisch mit dem Pumpenrotor.

Weitere vorteilhafte Massnahmen und bevorzugte Ausgestaltungen der Erfindung ergeben sich aus den abhängigen Ansprüchen.

Im Folgenden wird die Erfindung anhand von Ausführungsbeispielen und anhand der Zeichnungnäher erläutert. In der schematischen, nicht massstäblichen Zeichnung zeigen:
- Fig. 1:: eine schematische Darstellung eines ersten Ausführungsbeispiels des erfindungsgemässen Drehantriebs,
- Fig. 2:: eine schematische Darstellung eines zweiten Ausführungsbeispiels wie auf Seite 5 der Beschreibung, und
- Fig. 3:: eine schematische Darstellung einer Blutpumpe mit einem erfindungsgemässen Drehantrieb.

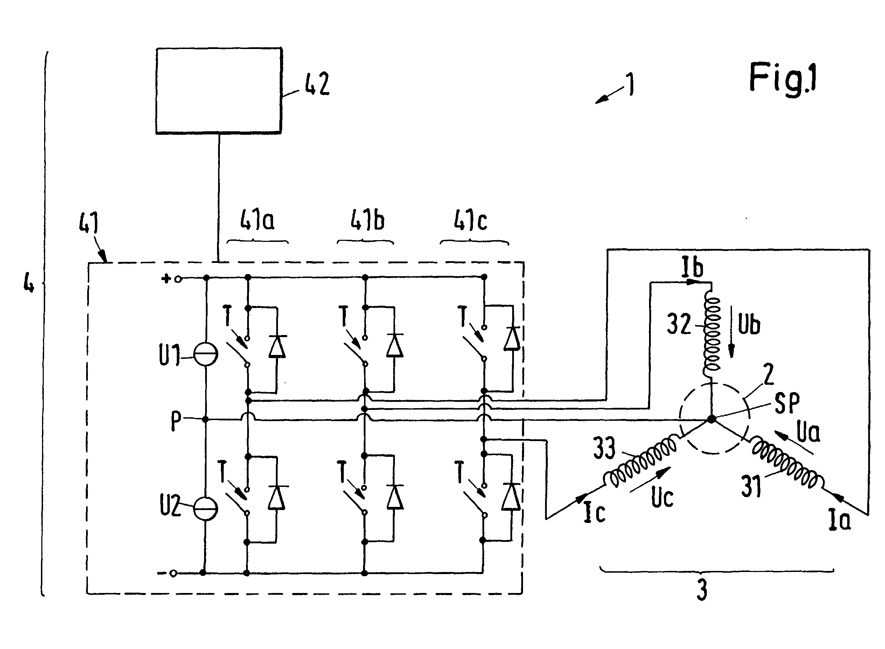

Fig. 1 zeigt in einer schematischen Darstellung ein erstes Ausführungsbeispiel des erfindungsgemässen permanentmagnetisch erregten elektrischen Drehantriebs, der gesamthaft mit dem Bezugszeichen 1 bezeichnet ist. Der Drehantrieb 1 umfasst einen permanentmagnetischen Rotor 2, einen Stator 3 und eine Stelleinrichtung 4 mit einer Verstärkereinheit 41 und einer Regeleinheit 42.

Der Stator 3 weist eine Antriebswicklung auf, welche bei diesem Ausführungsbeispiel drei Stränge 31, 32, 33 hat, die jeweils zu einer anderen elektrischen Phase gehören, das heisst, die Antriebswicklung ist dreiphasig ausgestaltet. Mittels der Antriebswicklung ist ein magnetisches Drehfeld als Antriebsfeld erzeugbar, das ein Drehmoment auf den Rotor 2 bewirkt, wodurch dieser in Rotation versetzt wird.

Im Folgenden wird auf den für die Praxis besonders wichtigen Fall Bezug genommen, dass die Antriebswicklung dreiphasig mit den drei Strängen 31,32,33 ausgestaltet ist. Es versteht sich jedoch, dass der erfindungsgemässe Drehantrieb in sinngemäss gleicher Weise auch zweiphasig, das heisst mit einer Antriebswicklung, die zwei Stränge aufweist, oder n-phasig (mit n>3), das heisst mit einer Antriebswicklung, die n Stränge umfasst, ausgestaltet sein kann.

Die Antriebswicklung mit den drei Strängen 31,32,33 ist in an sich bekannter Weise auf den Stator 3 gewickelt und bildet somit mehrere diskrete Spulen zum Erzeugen des magnetischen Antriebsfelds. Dabei können mehrere diskrete Spulen elektrisch in einer Parallelschaltung oder in einer Serienschaltung miteinander verbunden sein. Die Gesamtheit aller der diskreten Spulen, die miteinander elektrisch parallel oder seriell verbunden sind, wird als ein Strang 31 oder 32 oder 33 der Antriebswicklung bezeichnet. Natürlich ist es auch möglich, dass jeder Strang 31,32,33 nur eine diskrete Spule umfasst.

Jeder Strang bildet mit dem ihn versorgenden Teil der Stelleinrichtung 4 eine separate elektrische Phase. Die Stelleinrichtung 4 kann jeden Strang 31,32,33 jeweils mit einem Phasenstrom la, Ib, Ic oder einer Phasenspannung Ua, Ub, Uc als Stellgrösse versorgen. Die Stelleinrichtung 4 kann also als Stromsteller oder als Spannungssteller für die Antriebswicklung ausgebildet sein.

Das erste Ausführungsbeispiel ist also ein dreiphasiger, permanentmagnetisch erregter Drehfeldmotor. Erfindungsgemäss umfasst die Stelleinrichtung 4 für jeden Strang 31,32,33 einen separaten Leistungsverstärker 41a, 41b, bzw. 41c, sodass die Stellgrösse Ia,Ib,Ic bzw. Ua,Ub,Uc für jeden Strang 31, 32 oder 33 unabhängig von der Stellgrösse für die anderen Stränge regelbar ist.

Bei dem ersten Ausführungsbeispiel sind die drei Leistungsverstärker 41a, 41b, 41c jeweils Brückenzweige einer Verstärkereinheit 41, die beispielsweise Teil eines dreiphasigen Drehstromstellers sind, von dem in Fig. 1 nur der Leistungsteil dargestellt ist. Die Signalverarbeitungs- und Regeleinrichtungen, die beispielsweise ganz oder teilweise in der als integrierte Schaltung ausgestalteten Verstärkereinheit 41 integriert sein können, sind in Fig. 1 in symbolischer Weise in der Regeleinheit 42 zusammengefasst.

Für jeden Strang 31, 32, 33 der Antriebswicklung ist jeweils ein Brückenzweig der Verstärkereinheit 41 als separater bipolarer Leistungsverstärker 41a, 41b bzw. 41c vorgesehen. Jeder Brückenzweig kann in an sich bekannter Weise mittels Schalttransistoren T und Freilaufdioden den zugehörigen Strang 31 oder 32 oder 33 mit dem jeweiligen Phasenstrom Ia,Ib,Ic oder der jeweiligen Phasenspannung Ua,Ub,Uc versorgen. Die Schalttransistoren T sind vorzugsweise Feldeffekttransistoren (FET). Die Verstärkereinheit 41 wird mit zwei Betriebspotentialen betrieben, die in Fig. 1 mit + und - bezeichnet sind. Diese Beriebspotentiale +,- sind Gleichspannungspotentiale. Für die Speisung der Verstärkereinheit 41 sind zwei stabilisierte Spannungsquellen U1 und U2 vorgesehen, die einen gemeinsamen Pol P aufweisen. Der gemeinsame Pol P liegt auf einem Potential, dass zwischen den beiden Betriebspotentialen + und - liegt, beispielsweise auf Erdpotential. Die eine Spannungsquelle U1 liefert dann die bezüglich des gemeinsamen Pols P positive Versorgungsspannung und die andere Spannungsquelle U2 die bezüglich des gemeinsamen Pols P negative Versorgungsspannung.

Jeder Strang 31,32,33 ist einerseits mit dem ihn versorgenden bipolaren Leistungsverstärker 41a, 41b, 41c verbunden. Andererseits ist jeder Strang mit einem gemeinsamen belastbaren Sternpunkt SP verbunden, der auf dem gleichen Potential liegt, wie der gemeinsame Pol P der Spannungsquellen U1, U2. Der Sternpunkt SP ist beispielsweise über eine elektrische Leitung direkt mit dem gemeinsamen Pol P der Spannungsquellen U1, U2 verbunden. Die drei Stränge 31,32,33 der Antriebswicklung sind also in einer Sternpunktschaltung geschaltet, wobei jedoch der Sternpunkt SP belastbar ist, das heisst mit einem belastbaren Potential verbunden ist, sodass, abgesehen von den drei Phasenströmen la,lb,lc ein zusätzlicher Strom über den Sternpunkt SP abfliessen bzw. in diesen hineinfliessen kann. Das bedeutet, die übliche Sternpunktbedingung, dass die Summe der Phasenströme la,lb,lc im Sternpunkt SP immer Null sein muss, ist bei dieser Schaltung nicht mehr notwendig. Dies hat zur Folge, dass jeder Phasenstrom Ia,Ib,Ic vollkommen unabhängig von den anderen Phasenströmen geregelt werden kann. Zur Bestimmung der einzelnen Phasenströme la,lb,lc können in den einzelnen Phasen jeweils Strommessgeräte (nicht dargestellt) vorgesehen sein.

Im normalen Betriebszustand, das heisst wenn alle drei Phasen fehlerfrei sind, arbeitet der dreiphasige Drehantrieb 1 in der an sich bekannten Weise. Die Drehzahl des Rotors 2 wird mittels eines Drehzahlreglers geregelt, der beispielsweise in der Regeleinheit 42 integriert ist. Ferner können Sensoren (nicht dargestellt) vorgesehen sein, aus deren Signal der Drehzahlregler die momentane Drehzahl des Rotors 2 ermittelt. Weicht die Drehzahl von einer vorgebbaren Soll-Drehzahl ab, so modifiziert der Drehzahlregler durch eine entsprechende Ansteuerung der Verstärkereinheit die einzelnen Phasenströme la,lb,lc oder die einzelnen Phasenspannungen Ua,Ub,Uc derart, dass der Rotor 2 seine Soll-Drehzahl erreicht.

Falls in einer der elektrischen Phasen ein Fehler auftritt, beispielsweise ein Unterbruch in einer elektrischen Leitung eines Strangs 31,32,33, so ist grundsätzlich keine Massnahme notwendig. Sobald nämlich der Drehzahlregler eine Abweichung der momentanen Rotordrehzahl von der Soll-Drehzahl detektiert, die beispielsweise daraus resultiert, dass einer der Stränge 31,32 oder 33 ausfällt, so erhöht der Drehzahlregler automatisch die Phasenströme bzw. die Phasenspannungen in den beiden fehlerfreien Strängen, sodass der Rotor wieder mit seiner Soll-Drehzahl läuft. Dies ist möglich, weil die einzelnen Phasenströme la,lb,lc bzw. die einzelnen Phasenspannungen Ua,Ub,Uc vollkommen unabhängig voneinander regelbar sind. Somit ist auch beim kompletten Ausfall einer Phase, die beispielsweise durch den Ausfall eines bipolaren Leistungsverstärkers 41a,41b,41c oder durch einen Leitungsdefekt in einem der Stränge 31,32,32 oder in der Verbindung zwischen einem Strang und dem zugehörigen Leistungsverstärker verursacht wird, eine ordnungsgemässe Arbeitsweise des Drehantriebs 1 mit den noch verbleibenden fehlerfreien Phasen gewährleistet.

Bei einer drei- oder mehrphasigen Ausgestaltung des Drehantriebs 1 kann dieser sogar bei einem Ausfall von zwei oder mehr Phasen noch ordnungsgemäss mit der verbleibenden fehlerfreien Phase bzw. den verbleibenden fehlerfreien Phasen betrieben werden. Die Minimalanforderung ist es, dass noch eine der Phasen fehlerfrei arbeitet. Falls nur noch eine der Phasen fehlerfrei ist, so arbeitet der Drehantrieb 1 als einphasiger Wechselstrommotor. Solange der Drehantrieb 1 in diesem Falle nicht angehalten wird, dreht er weiter, arbeitet also noch korrekt. Falls ein solcher einphasiger Wechselstrommotor jedoch angehalten wird, ist es je nach relativer Stellung von Rotor 2 und Stator 3 möglich, dass der Drehantrieb 1 nicht mehr angefahren werden kann. Dem kann jedoch abgehalfen werden, indem der Stator 3 so ausgestaltet wird, dass der Rotor 2 beim Anhalten in einer vorgegebenen "Raststellung" stehen bleibt, die so gewält wird, dass der Rotor 2 aus dieser Raststellung wieder anlaufen kann. Diese Massnahme ist von einphasigen Wechselstrommotoren hinreichend bekannt und wird daher hier nicht näher erläutert.

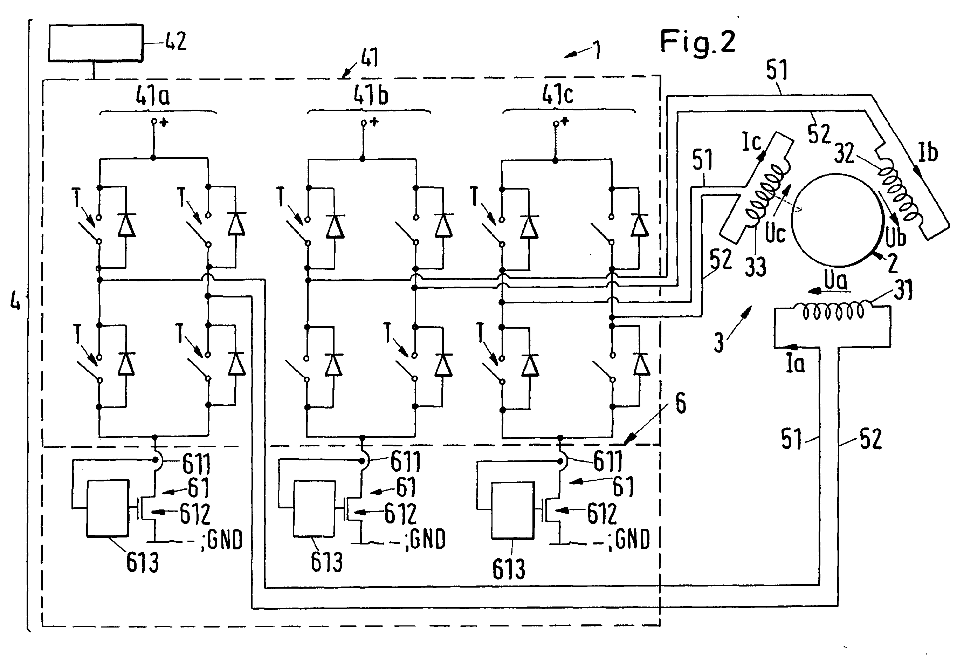

Fig. 2 zeigt in einer schematischen Darstellung ein zweites wie auf Seite 5 der Beschreibung Ausführungsbeispiel, wobei identische oder von der Funktion her gleichwertige Teile mit den gleichen Bezugszeichen versehen sind wie in Fig. 1. Im Folgenden werden die Unterschiede zum ersten Ausführungsbeispiel beschrieben, ansonsten gelten die Ausführungen bezüglich des ersten Ausführungsbeispiels in sinngemäss gleicher Weise auch für das zweite Ausführungsbeispiel.

Bei dem zweiten Ausführungsbeispiel weist jeder Strang 31,32,33 der Antriebswicklung des Stators 3 jeweils zwei elektrische Anschlussleitungen 51 und 52 auf, wobei die Anschlussleitungen 51,52 eines Strangs jeweils getrennt und unabhängig von den Anschlussleitungen 51,52 der anderen Stränge sind. Im Unterschied zum ersten Ausführungsbeispiel ist also kein gemeinsamer Sternpunkt vorgesehen, mit dem jeder Strang 31,32,33 der Antriebswicklung verbunden ist, sondern für jeden Strang werden jeweils beide Anschlussleitungen 51,52 aus dem Stator 3 herausgeführt und getrennt mit den Leistungsverstärkern 41 a, 41 b, 41 c der Verstärkereinheit 41 verbunden. Somit ist jeder Leistungsverstärker 41 a, 41 b, 41 c mit genau zwei Anschlussleitungen 51,52 verbunden, die zu demselben Strang 31 oder 32 oder 33 gehören.

Wie dies Fig. 2 zeigt, sind die einzelnen bipolaren Leistungsverstärker 41 a,41 b,41 c für die Stränge 31,32,33 jeweils als H-Brückenschaltung ausgestaltet, wobei für jede Phase bzw. für jeden Strang 31,32,33 der Antriebswicklung ein separater Leistungverstärker vorgesehen ist, sodass eine unabhängige Regelung der einzelnen Phasenströme Ia,Ib,Ic bzw. der einzelnen Phasenspannungen Ua,Ub,Uc möglich ist. Die H-Brückenschaltungen sind in an sich bekannter Weise mit Schalttransistoren T und Freilaufdioden realisiert und werden mit den. Das Betriebspotential - ist beispielsweise das Erdpotential GND. Betriebspotentialen + und - betrieben. Das Betriebspotential - ist beispielsweise das Erdpotential GND. Die Schalttransistoren T sind vorzugsweise Feldeffekttransistoren (FET) und insbesondere Leistungs-MOS-FET's.

Die prinzipielle Funktionsweise des zweiten Ausführungsbeispiels ist die gleiche wie die des ersten Ausführungsbeispiels.

Auch bei dem zweiten Ausführungsbeispiel brauchen grundsätzlich keine besonderen Massnahmen getroffen zu werden, um den Drehantrieb 1 beim Ausfall einer Phase oder mehrerer Phasen mit den verbleibenden fehlerfreien Phasen bzw. der verbleibenden fehlerfreien Phase weiter zu betreiben. Insbesondere kann der Drehantrieb auch dann noch weiter betrieben werden, wenn in einer Phase, z. B in einem Strang 31,32,33 oder in einem Schalttransistor T des zugehörigen Leistungsverstärkers 41a,41b,41c, ein Kurzschluss auftritt. Da jedoch das Fliessen von Kurzschlussstömen auch einen unnötigen und unnützen Energieverbrauch bedeutet, ist vorzugsweise ein Überwachungsmodul 6 vorgesehen, welche beim Auftreten eines Fehlers, insbesondere eines Kurzzschlusses, in einer der elektrischen Phasen diese Phase deaktiviert. Das Überwachungsmodul 6 umfasst für jede Phase eine Überstromsicherung 61. Wie dies Fig. 2 zeigt, ist bei jedem Leistungsverstärker 41a,41b,41c zwischen der H-Brückenschaltung und einem der Betriebspotentiale +,- jeweils eine Überstromsicherung 61 zwischengeschaltet. Vorzugsweise ist die Überstromsicherung 61 seriell zwischen der H-Brückenschaltung und dem Betriebspotential - vorgesehen, welches hier das Erdpotential GND ist.

Die Überstromsicherung 61 umfasst ein Strommessgerät 611, ein Schaltelement 612, das vorzugsweise ein FET ist, sowie eine Überstromabschaltung 613. Die Überstromabschaltung 613 überwacht mittels des Strommessgeräts 611 die Stärke des fliessenden Stroms. Falls dieser einen vorgebbaren Grenzwert überschreitet, öffnet die Überstromabschaltung 613 das Schaltelement 612, wodurch die zugehörige Phase abgeschaltet wird.

Die Überstromsicherung 61 kann alternativ auch jeweils eine schnelle Sicherung, beispielsweise eine Schmelzsicherung, sein, die in Serie zwischen der H-Brücke und einem der Betriebspotentiale +,- angeordnet ist.

Eine Überstromsicherung kann selbstverständlich auch in sinngemäss gleicher Weise in die Brückenzweige des in Fig. 1 dargestellten Ausführungsbeispiels eingebaut werden.

Alternativ oder ergänzend kann das Überwachungsmodul 6 auch andere Elemente umfassen, um beim Auftreten eines Fehlers in einer Phase diese Phase abzuschalten. Beispielsweise können in dem Überwachungsmodul 6 Mittel vorgesehen sein, um beim Auftreten eines Fehlers in einer Phase die Schalttransistoren T des zugehörigen Leistungsverstärkers 41a oder 41b oder 41c so zu betätigen, dass in dieser Phase kein Strom mehr fliesst (Öffnen der Schalter). Das Überwachungsmodul 6 kann ferner Mittel zum Detektieren von Fehlern aufweisen.

Abweichend von den beiden beschriebenen Ausführungsbeispielen sind natürlich auch noch andere Ausgestaltungen der Verstärkereinheit 41 möglich, beispielsweise andere Formen von Schaltverstärkern oder Analogverstärker. Wichtig ist jedoch, dass die Verstärkereinheit 41 so ausgebildet ist, dass sie bipolar betreibbar ist, womit gemeint ist, dass sowohl die Phasenströme als auch die Phasenspannungen positives und negatives Vorzeichen annehmen können. Zusätzlich muss die Verstärkereinheit 41 jede Phase mit jeweils einer Stellgrösse (Phasenstrom la,lb,lc bzw. Phasenspannung Ua,Ub,Uc) versorgen können, die unabhängig von den Stellgrössen für die anderen Phasen regelbar ist.

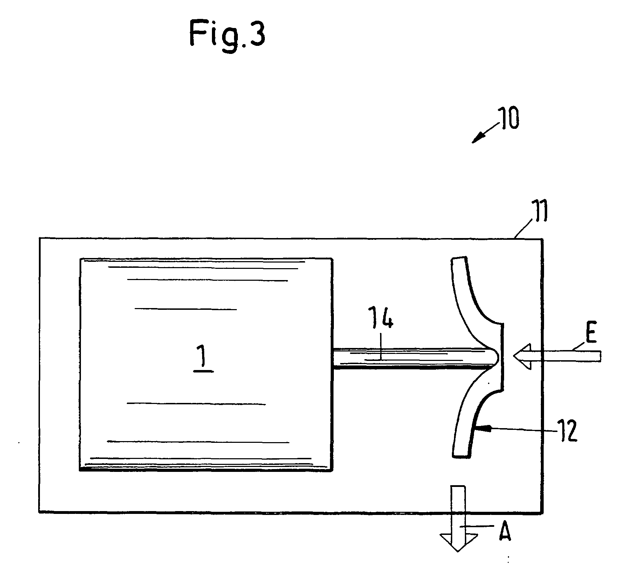

Fig. 3 zeigt stark schematisiert eine Blutpumpe 10, die mit einem erfindungsgemässen Drehantrieb 1 betrieben wird. Die Blutpumpe 10 umfasst ein Gehäuse 11, in welchem ein Pumpenrotor 12 vorgesehen ist, der als Laufrad mit mehreren Flügeln oder Schaufeln ausgebildet ist. Der Pumpenrotor 12 ist auf einer Achse 14 angeordnet und drehfest mit dieser verbunden. Die Achse 14 wird vom Rotor 2 des Drehantriebs 1 angetrieben. Die Blutpumpe 10 ist hier als Zentrifugalpumpe ausgebildet. Das zu fördernde Blut gelangt durch einen mit dem Blutkreislauf verbundenen Eingang zum Pumpenrotor 12 wie dies der Pfeil E in Fig. 3 andeutet und wird von dem sich drehenden Pumpenrotor 12 zum Ausgang gefördert. Durch den Ausgang gelangt das Blut zurück in den Blutkreislauf wie dies der Pfeil A in Fig. 3 andeutet.

Die Blutpumpe 10 mit dem Drehantrieb 1 kann beispielsweise nach dem Prinzip der Spaltrohrpumpe bzw. des Spaltrohrmotors ausgestaltet sein.

Die Achse 14 und/oder der Rotor 2 des Drehantriebs 1 können mittels mindestens einer magnetischen Lagervorrichtung gelagert sein. Dazu kann eine separate, das heisst von dem Drehantrieb 1 verschiedene magnetische Lagervorrichtung für den Rotor 2 bzw. die Achse 14 vorgesehen sein. Es ist aber auch möglich, dass die magnetische Lagervorrichtung in den Drehantrieb 1 integriert ist. Dazu kann der Drehantrieb 1 als lagerloser Motor ausgestaltet sein, so wie dies beispielsweise in der WO-A-96/31934 offenbart ist. Der lagerlose Motor verdankt seinen Namen der Tatsache, dass der Stator 3 als Antriebs- und Lagerstator ausgestaltet ist, der den Rotor 2 sowohl magnetisch lagert als auch antreibt. Das bedeutet, bei einem lagerlosen Motor bilden das Magnetlager und der Drehantrieb eine nicht trennbare körperliche Einheit. Beim lagerlasen Motor umfasst der Stator 3 neben der Antriebswicklung ferner eine Steuerwicklung. Mit diesen beiden Wicklungen ist ein magnetisches Drehfeld generierbar, welches zum einen ein antreibendes Drehmoment auf den Rotor 2 ausübt, und welches zum anderen eine beliebig einstellbare Querkraft auf den Rotor 2 ausübt, sodass dessen radiale Position aktiv regelbar ist. Bezüglich der axialen Richtung und bezüglich Verkippungen relativ zur Drehachse ist der permanentmagnetisch erregte Rotor 2 passiv magnetisch, das heisst nicht ansteuerbar durch Reluktanzkräfte stabilisiert. Bezüglich weiterer Details sei hier auf die WO-A-96/31934 verwiesen. Es können jedoch noch zusatzliche Lager, wie beispielsweise magnetische Axiallager oder hydrodynamische Lager für die Lagerung des Rotors 2 des Drehantriebs 1 bzw. die Achse 14 vorgesehen sein.

Eine weitere Variante für die Blutpumpe 10 besteht darin, den Rotor 2 des Drehantriebs 1 als Pumpenrotor zum Fördern des Bluts auszugestalten. Bei dieser Ausgestaltung, die in der WO-A-96/31934 als Integralrotor bezeichnet wird, ist der Pumpenrotor 12 identisch mit dem Rotor 2 des Drehantriebs 1. Bezüglich konkreter Beispiele, wie ein solcher Rotor 2,12 ausgebildet sein kann, wird auf die WO-A-96/31934 verwiesen. Dadurch, dass der Rotor 2 des Drehantriebs als Pumpenrotor mit Flügeln oder Schaufeln zum Fördern des Bluts ausgestaltet ist, lässt sich eine besonders platzsparende und kompakte Blutpumpe realisieren.

Für eine besonders sichere Blutpumpe 10 ist es vorteilhaft, wenn ausser dem fehlertoleranten Drehantrieb 1 eine ebenfalls fehlertolerante magnetische Lagervorrichtung für den Rotor 2 bzw. die Achse 14 vorgesehen ist. Dies gilt sowohl für den Fall, dass die magnetische Lagervorrichtung nach dem Prinzip des lagerlosen Motors in den Drehantrieb 1 integriert ist als auch für den Fall, dass mindestens eine separate, das heisst vom Drehantrieb 1 verschiedene magnetische Lagervorrichtung für den Rotor 2 bzw. die Achse 14 vorgesehen ist.

Eine solche fehlertolerante magnetische Lagervorrichtung wird in der europäischen Patentanmeldung Nr. 98810956.7 offenbart. Diese fehlertolerante magnetische Lagervorrichtung umfasst einen Stator (den Lagerstator) mit einer mindestens drei Stränge aufweisenden Steuerwicklung zum Erzeugen eines magnetisches Steuerfelds, mit welchem die Position des Rotors 2 bzw. der Achse 14 bezüglich des Lagerstators regelbar ist, wobei jeder Strang zu einer anderen elektrischen Phase gehört, sowie eine Stelleinrichtung, die in einem ersten Betriebsmodus jeden Strang mit jeweils einem Phasenstrom oder jeweils einer Phasenspannung als Stellgrösse versorgt. Es sind Mittel vorgesehen, um die Stellgrösse für jeden Strang unabhängig von der Stellgrösse für die anderen Stränge zu regeln. Ferner ist eine Überwachungseinheit vorgesehen, welche die Lagervorrichtung in einen zweiten Betriebsmodus umschalten kann, in welchem eine reduzierte Anzahl von Phasen, die mindestens zwei ist, das magnetische Steuerfeld erzeugt.

Das magnetische Steuerfeld ist ein magnetisches Drehfeld.

Die magnetische Lagervorrichtung gemäss der europäischen Patentanmeldung Nr. 98810956.7 kann entweder nach dem Prinzip des lagerlosen Motors ausgebildet sein oder als separate magnetische Lagervorrichtung. Im erstgenannten Fall ist der Lagerstator identisch mit dem Stator 3 des Drehantriebs 1, der folglich als Antriebs- und Lagerstator ausgebildet ist. Das heisst neben der mindestens zweiphasigen Antriebswicklung ist ferner die mindestens dreiphasige Steuerwicklung auf dem gleichen Stator vorgesehen.

Die fehlertolerante Lagervorrichtung kann in zwei Betriebsmodi betrieben werden. Im ersten Betriebsmodus wird das magnetische Steuerfeld mit allen Phasen der drei- oder höherphasigen Steuerwicklung, also mit allen ihren Strängen, generiert. Im zweiten Betriebsmodus wird das magnetische Steuerfeld nur noch mit einer reduzierten Anzahl von Phasen, die jedoch mindestens zwei beträgt, erzeugt. Es werden also nicht mehr alle Stränge der Steuerwicklung verwendet, einer oder mehrere Stränge der Steuerwicklung sind in diesem zweiten Betriebsmodus "abgeschaltet". Eine zweiphasige Steuerwicklung reicht nämlich aus, um das für die Regelung der radialen Position des Rotors 2 bzw. der Achse 14 bezüglich des Lagerstators benötigte Steuerfeld zu generieren. Dazu ist es jedoch notwendig, dass die beiden Phasenströme oder Phasenspannungen unabhängig voneinander geregelt werden können.

Durch die beiden Betriebsmodi ist es möglich, beim Auftreten eines Fehlers in einer der Phasen, beispielsweise beim Ausfall eines Wicklungsstrangs aufgrund eines Leitungsbruchs oder eines Kurzschlusses oder beim Ausfall des Verstärkers, der diese Phase versorgt, die Lagervorrichtung mit einer reduzierten Anzahl von Phasen weiterhin zu betreiben, ohne dass Zugeständnisse an das ordnungsgemässe Funktionieren der magnetischen Lagerung vonnöten sind. Diese Fehlertoleranz, nämlich auch beim Ausfall einer Phase der Steuerwicklung noch einen sicheren Betrieb der magnetischen Lagervorrichtung zu gewährleisten, bedeutet eine enorme Steigerung der Betriebssicherheit im Vergleich zu anderen Magnetlagern.

Vorzugsweise überwacht die Überwachungseinheit das Funktionieren jeder einzelnen Phase und schaltet beim Auftreten eines Fehlers in einer Phase die Lagervorrichtung in den zweiten Betriebsmodus um, in welchem nur noch die Phasen ohne Fehler das magnetische Steuerfeld erzeugen.

Wie bereits erwähnt ist es für den Fall, dass die Steuerwicklung nur noch zweiphasig betrieben wird, notwendig, dass der Phasenstrom oder die Phasenspannung in den beiden Strängen der Steuerwicklung unabhängig voneinander regelbar sind. Üblicherweise werden die Phasenströme oder die Phasenspannungen für die einzelnen Phasen von einer Verstärkereinheit bereitgestellt, die in der Stelleinrichtung enthalten ist.

Bei einer ersten Ausführungsform der fehlertoleranten Lagervorrichtung umfassen die Mittel zum unabhängigen Regeln der Stellgrösse (Phasenstrom oder Phasenspannung) einen belastbaren Sternpunkt, mit dem jede Phase verbunden ist, und der auf ein Potential gelegt ist, welches zwischen den beiden Betriebspotentialen der Verstärkereinheit liegt. Bei üblichen dreiphasigen Steuerwicklungen sind die drei Stränge jeweils mit einem gemeinsamen Sternpunkt verbunden, wobei die Bedingung erfüllt sein muss, dass die Summe der Phasenströme im Sternpunkt Null ist. Durch die Massnahme, den Sternpunkt belastbar zu machen, das heisst ihn auf ein belastbares Potential zu legen, kann diese Bedingung aufgehoben werden, sodass jeder Phasenstrom bzw. jede Phasenspannung unabhängig von den anderen regelbar ist.

Bei einer zweiten Ausführungsform umfasst die Verstärkereinheit als Mittel zum unabhängigen Regeln der Stellgrösse (Phasenstrom oder Phasenspannung) einen separaten Leistungsverstärker für jede Phase, der als H-Brückenschaltung ausgestaltet sind. Auch diese Massnahme ermöglicht ein unabhängiges Regeln der einzelnen Phasenströme oder Phasenspannungen für die Lagervorrichtung.

Gemäss einem weiterführenden Aspekt hat die fehlertolerante magnetische Lagervorrichtung mindestens drei Positionssensoren zum Bestimmen der radialen Position des Rotors 2 bzw. der Achse 14 in einem Statorsystem, das bezüglich des Lagerstators ortsfest ist. Da prinzipiell zwei Positionssensoren ausreichen, um die radiale Position des Rotors bzw. der Achse im Statorsystem zu bestimmen, lässt sich durch die Verwendung von mindestens drei Positionssensoren eine Fehlertoleranz der Positionssensorik, welche wesentlich für die Regelung der Position des zu lagernden Körpers und damit den Betrieb der Lagervorrichtung ist, erreichen. Beim Ausfall eines Positionssensors ist nämlich die radiale Position des Körpers immer noch eindeutig mittels der verbleibenden Positionssensoren möglich. Dies bedeutet eine weitere Erhöhung der Betriebssicherheit der magnetischen Lagervorrichtung.

Vorzugsweise ist eine Positionseinheit vorgesehen, welche die Signale der Positionssensoren mittels einer Transformation in ein zweikomponentiges Positionssignal umwandelt, dessen eine Komponente die X-Koordinate und dessen andere Komponente die Y-Koordinate des zu lagernden Körpers im Statorsystem repräsentiert.

Bei einer bevorzugten Ausführungsform sind die drei Positionssensoren so angeordnet, dass jeweils zwei benachbarte Positionssensoren bezüglich der Umfangsrichtung des Lagerstators um einen Winkel von 120° zueinander versetzt sind. Bei dieser Anordnung ist es nämlich auf besonders einfache Weise möglich, den Ausfall eines Positionssensors zu kompensieren. Falls der defekte Positionssensor das Signal Null liefert, ist prinzipiell überhaupt keine Massnahme zur Kompensation notwendig. Die Transformation in das zweikomponentige Positionssignal kann mit unveränderter Transformationsmatrix durchgeführt werden und führt dennoch zu einer korrekten Positionsregelung.

Vorzugsweise überwacht die Positionseinheit das Funktionieren der Positionssensoren. Dies kann beispielsweise so erfolgen, dass die Positionseinheit für jeden Positionssensor überprüft, ob das von ihm gelieferte Signal oder der Mittelwert mehrerer seiner Signale innerhalb eines vorgebbaren Toleranzbereichs liegt. Auch ist es möglich, dass die Positionseinheit mittels des Signals von mindestens einem der Positionssensoren die anderen Positionssensoren überwacht.

Detektiert die Positionseinheit bei der oben erwähnten 120°-Anordnung der Positionssensoren das Auftreten eines Fehlers, so setzt sie das Signal des zugehörigen Positionssensors vor der Transformation auf Null und führt dann die Transformation mit der gleichen Transformationsmatrix durch wie im Falle, dass alle Positionssensoren korrekt arbeiten.

Falls der Winkel zwischen benachbarten Positionssensoren nicht 120° beträgt, so ist im allgemeinen beim Ausfall eines Positionssensors eine Änderung der Transformation notwendig, um eine korrekte Positionsregelung zu gewährleisten.

Im Falle des Auftretens eines Fehlers bei einem Positionssensor kann dann die Positionseinheit eine andere Transformation zur Bestimmung des Positionssignals auswählen, in welche nur die Signale von fehlerfreien Positionssensoren eingehen.

Bezüglich weiterer Details einer solchen fehlertoleranten magnetischen Lagervorrichtung sei auf die europäische Patentanmeldung Nr. 98810956.7 verwiesen.

Falls der permanentmagnetisch erregte elektrische Drehantrieb 1 und das fehlertolerante Magnetlager gemeinsam als lagerloser Motor ausgebildet sind, ist der Rotor 2 vorzugsweise scheiben- oder ringförmig ausgestaltet.

## Patentansprüche

1. Permanentmagnetisch erregter elektrischer Drehantrieb für eine Blutpumpe, mit einem permanentmagnetischen Rotor (2) und einem Stator (3), welcher Stator (3) eine mindestens zwei Stränge (31,32,33) aufweisende Antriebswicklung zum Erzeugen eines magnetischen Antriebsfelds umfasst, das ein Drehmoment auf den Rotor (2) bewirkt, wobei jeder Strang (31,32,33) zu einer anderen elektrischen Phase gehört, ferner mit einer Stelleinrichtung (4), die jeden Strang (31,32,33) jeweils mit einem Phasenstrom (Ia,Ib,Ic) oder jeweils mit einer Phasenspannung (Ua,Ub,Uc) als Stellgrösse versorgt, wobei die Stelleinrichtung (4) für jeden Strang einen separaten Leistungsverstärker (41 a,41 b,41 c) umfasst, sodass die Stellgrösse für jeden Strang (31,32,33) unabhängig von der Stellgrösse für die anderen Stränge regelbar ist, **dadurch gekennzeichnet, dass** ein belastbarer Sternpunkt (SP) vorgesehen ist, der mit jedem Strang (31,32,33) der Antriebswicklung verbunden ist und über eine elektrische Leitung direkt mit einem stets gleichen Potential verbunden ist.

2. Drehantrieb nach Anspruch 1 mit zwei stabilisierten Spannungsquellen (U1,U2) zur Versorgung der Stelleinrichtung (4), wobei die Spannungsquellen (U1,U2) einen gemeinsamen Pol (P) aufweisen und wobei der belastbare Sternpunkt (SP) auf dem gleichen Potential liegt wie der gemeinsame Pol (P) der Spannungsquellen (U1,U2).

3. Drehantrieb nach einem der vorangehenden Ansprüche mit einem Überwachungsmodul (6), welches derart ausgestaltet ist, dass es beim Auftreten eines Fehlers in einer der elektrischen Phasen diese deaktivieren kann.

4. Drehantrieb nach einem der vorangehenden Ansprüche, bei welcher für jede elektrische Phase eine Überstromsicherung (61) vorgesehen ist.

5. Drehantrieb nach einem der vorangehenden Ansprüche, bei welchem der Drehantrieb (1) als lagerloser Motor ausgebildet ist, der den Rotor (2) des Drehantriebs (1) magnetisch lagert, wobei der Stator (3) als Antriebs- und Lagerstator ausgestaltet ist, der ausser der Antriebswicklung ferner eine Steuerwicklung umfasst.

6. Blutpumpe mit einem permanentmagnetisch erregten elektrischen Drehantrieb (1) gemäss einem der vorangehenden Ansprüche.

7. Blutpumpe nach Anspruch 6, wobei der Rotor (2) des Drehantriebs als Pumpenrotor zum Fördern des Bluts ausgestaltet ist.

8. Blutpumpe nach Anspruch 6 oder 7 mit einer fehlertoleranten magnetischen Lagervorrichtung für einen Rotor (2) oder für eine Achse (14), welche Lagervorrichtung einen Lagerstator umfasst mit einer mindestens drei Stränge aufweisenden Steuerwicklung zum Erzeugen eines magnetisches Steuerfelds, mit welchem die Position des Rotors (2) bzw. der Achse (14) bezüglich des Lagerstators regelbar ist, wobei jeder Strang zu einer anderen elektrischen Phase gehört, sowie eine Stelleinrichtung, die in einem ersten Betriebsmodus jeden Strang mit jeweils einem Phasenstrom oder jeweils einer Phasenspannung als Stellgrösse versorgt, wobei Mittel vorgesehen sind, um die Stellgrösse für jeden Strang unabhängig von der Stellgrösse für die anderen Stränge zu regeln, sowie eine Überwachungseinheit, welche derart ausgestaltet ist, dass sie die Lagervorrichtung in einen zweiten Betriebsmodus umschalten kann, in welchem eine reduzierte Anzahl von Phasen, die mindestens zwei ist, das magnetische Steuerfeld erzeugt.

## Claims

1. A permanent magnetically excited electrical rotary drive for a blood pump having a permanent magnetic rotor (2) and a stator (3), said stator (3) including a drive winding having at least two loops (31, 32, 33) for the production of a magnetic drive field which produces a torque on the rotor (2), with each loop (31, 32, 33) belonging to a different electrical phase, furthermore including a setting device (4) which supplies each loop (31, 32, 33) in each case with a phase current (Ia, Ib, Ic) or in each case with a phase voltage (Ua, Ub, Uc) as a setting parameter, wherein the setting device (4) includes a separate power amplifier (41a, 41b, 41c) for each loop so that the setting parameter for each loop (31, 32, 33) can be regulated independently of the setting parameter for the other loops, **characterized in that** a loadable star point (SP) is provided which is connected to each loop (31, 32, 33) of the drive winding and is connected via an electrical line directly to a potential which is always the same.

2. A rotary drive in accordance with claim 1 having two stabilized voltage sources (U1, U2) for supplying the setting device (4), wherein the voltage sources (U1, U2) has a common pole (P) and wherein the loadable star point (SP) lies at the same potential as the common pole (P) of the voltage sources (U1, U2).

3. A rotary drive in accordance with any one of the preceding claims having a monitoring module (6) which is made such that it can deactivate one of the electrical phases when a fault arises therein.

4. A rotary drive in accordance with any one of the preceding claims, in which an excessive current safety device (61) is provided for each electrical phase.

5. A rotary drive in accordance with any one of the preceding claims, in which the rotary drive (1) is made as a bearingless motor which magnetically journals the rotor (2) of the rotary drive (1), with the stator (3) being designed as a drive and bearing stator which furthermore includes a control winding in addition to the drive winding.

6. A blood pump having a permanent magnetically excited electrical rotary drive (1) in accordance with any one of the preceding claims.

7. A blood pump in accordance with claim 6, wherein the rotor (2) of the rotary drive is designed as a pump rotor for the forwarding of the blood.

8. A blood pump in accordance with claim 6 or claim 7 having a fault-tolerant magnetic bearing apparatus for a rotor (2) or for an axis (14), said bearing apparatus including a bearing stator with a control winding having at least three loops for producing a magnetic control field with which the position of the rotor (2) or of the axis (14) respectively can be regulated with respect to the bearing stator, with each loop belonging to a different phase, as well as a setting device which in a first operating mode provides each loop with in each case a phase current or with in each case a phase voltage as the setting parameter, with means being provided to regulate the setting parameter for each loop independently of the setting parameter for the other loops, as well as a monitoring unit which is designed to be able to switch the bearing apparatus into a second operating mode in which a reduced number of phases, which is at least two, produces the magnetic control field.

## Revendications

1. Entraînement électrique en rotation (1) à excitation par aimants permanents pour une pompe de sang, avec un rotor (2) à aimants permanents et un stator (3), ledit stator (3) comprenant au moins un enroulement d'entraînement présentant deux conducteurs de phase (31, 32, 33) pour produire un champ d'entraînement magnétique qui provoque un couple de rotation sur le rotor (2), où chaque conducteur de phase (31, 32, 33) appartient à une autre phase électrique, en outre avec une installation de réglage (4) qui alimente chaque conducteur de phase (31, 32, 33) respectivement en un courant de phase (la, lb, lc) ou respectivement en une tension de phase (Ua, Ub, Uc) comme grandeur de réglage, où l'installation de réglage (4) pour chaque conducteur de phase comprend un amplificateur de puissance séparé (41a, 41b, 41c) de sorte que la grandeur de réglage pour chaque conducteur de phase (31, 32, 33) est réglable indépendamment de la grandeur de réglage des autres conducteurs de phase, **caractérisé en ce qu'**un point neutre (SP) apte à être chargé est prévu, qui est relié à chaque conducteur de phase (31, 32, 33) de l'enroulement d'entraînement, et est relié par une ligne électrique directement à un potentiel qui est toujours le même.

2. Entraînement en rotation selon la revendication 1, avec deux sources de tension stabilisées (U1, U2) pour l'alimentation de l'installation de réglage (4), où les sources de tension (U1, U2) présentent un pôle commun (P), et où le point neutre (SP) apte à être chargé se situe au même potentiel que le pôle commun (P) des sources de tension (U1, U2).

3. Entraînement en rotation selon l'une des revendications précédentes, avec un module de surveillance (6) qui est réalisé de façon que lors de la survenue d'une erreur dans une des phases électriques, il puisse désactiver celle-ci.

4. Entraînement en rotation selon l'une des revendications précédentes, où est prévu pour chaque phase électrique une sécurité de surintensité (61).

5. Entraînement en rotation selon l'une des revendications précédentes, où l'entraînement en rotation (1) est réalisé comme moteur sans palier qui loge le rotor (2) de l'entraînement en rotation (1) d'une manière magnétique, où le stator (3) est réalisé comme stator d'entraînement et de palier qui, à part l'enroulement d'entraînement, comprend en outre un enroulement de commande.

6. Pompe de sang avec un entraînement en rotation électrique (1) excitée par des aimants permanents selon l'une des revendications précédentes.

7. Pompe de sang selon la revendication 6, où le rotor (2) de l'entraînement en rotation est réalisé comme rotor de pompe pour le convoyage du sang.

8. Pompe de sang selon la revendication 6 ou 7, avec un dispositif de palier magnétique tolérant aux erreurs pour un rotor (2) ou pour un axe (14), ledit dispositif de palier comprenant un stator de palier avec un enroulement de commande présentant au moins trois conducteurs de phase pour la production d'un champ de commande magnétique, au moyen duquel la position du rotor (2) respectivement de l'axe (14) est réglable par rapport au stator de palier, où chaque conducteur de phase appartient à une autre phase électrique, et une installation de réglage qui, dans un premier mode de fonctionnement, alimente chaque conducteur de phase respectivement avec un courant de phase ou respectivement une tension de phase comme grandeur de réglage, où des moyens sont prévus pour régler la grandeur de réglage pour chaque conducteur de phase indépendemment de la grandeur de réglage des autres conducteurs de phase, ainsi qu'une unité de surveillance qui est réalisée de façon qu'elle puisse commuter le dispositif de palier dans un deuxième mode de fonctionnement dans lequel un nombre réduit de phases qui est au moins de deux, produit le champ de commande magnétique.
